# EUROPEAN PATENT APPLICATION

(11) **EP 4 311 828 A1**
(43) Date of publication of application: **31.01.2024**
(21) Application number: 22186856.5
(22) Date of filing: 26.07.2022
(51) Int. Cl.: C07D 213/61, C07D 213/73

(54) **A PROCESS FOR THE PHOTOLYTIC CHLORINATION OF 3-HALOPYRIDINE WITH MOLECULAR CHLORINE**

(71) Applicant: BASF SE, 67056 Ludwigshafen am Rhein (DE); Lianhe Chemical Technology Co., Ltd., Yancheng, Jiangsu 224631 (CN)
(72) Inventor: Rack, Michael, 67056 Ludwigshafen an Rhein (DE); Xie, Enrique, Shanghai (CN); Dang, Weijie, Shanghai (CN); Li, Dave, Shanghai (CN); Yi, Kaiqiang, Shanghai (CN); Kaduskar, Rahul, 400705 Navi Mumbai (IN); Gockel, Birgit, 67056 Ludwigshafen an Rhein (DE); Shinde, Harish, 400705 Navi Mumbai (IN); Goetz, Roland, 67056 Ludwigshafen an Rhein (DE); Knoll, Daniel Maximilian, 67056 Ludwigshafen an Rhein (DE)
(74) Representative: BASF IP Association

(57) **Abstract**

The present invention relates to a process for the chlorination of 3-halopyridine with molecular chlorine in the presence of water under UV light irradiation in the vapor phase. The present invention further relates to a multi-step process for producing 5-chloro-3,6-difluoropyridin-2-amine.

## Description

The present invention relates to a process for the chlorination of 3-halopyridine with molecular chlorine in the presence of water under UV light irradiation in the vapor phase. The present invention further relates to a multi-step process for producing 5-chloro-3,6-difluoropyridin-2-amine (V).

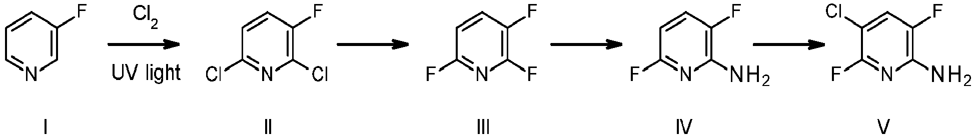

Compound V is a key intermediate and building block in the synthesis of uracil pyridines, which were described in WO 2017/202768 as herbicides.

Substituted pyridines are highly desirable intermediates in chemical syntheses, especially in life sciences, for example in the pharmaceutical or agrochemical industry. Hence, there is a constant demand for versatile and efficient procedures, which enable the direct functionalisation of the pyridine ring with halogen substituents. Such transformations are of particular interest for industrial scale processes, which, for economic reasons, ask for cheap and readily available reactants, such as molecular chlorine. Pyridine is generally known for its poor reactivity towards molecular chlorine or other electrophiles like bromine or nitric acid in aromatic electrophilic substitution reactions. This behaviour is caused by its relative electron deficiency as compared to, for example, the unsubstituted or π-donor-substituted benzene ring (Organikum, 20. Auflage, 1996, S. 337-340). Its reactivity is further reduced in the presence of electron-withdrawing substituents attached to the pyridine ring. This chemical behaviour has prompted chemists to resort to other, non-conventional procedures in order to gain access to these structures: for example, the preparation of intermediate compound III was described by Bobbio et al. (Chem. Eur. J. 2005, 11, 1903-2910). The synthesis starts from pentahalogenated pyridine VI. It involves the site-selective, reductive removal of chlorine and fluorine atoms using complex hydrides and palladium catalyzed hydrogenations.

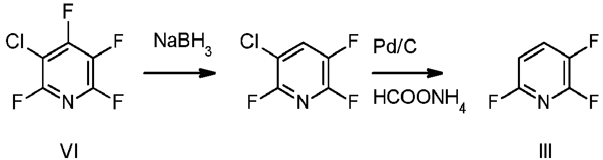

The starting material VI and the reactants are expensive, the transformations lack atom-efficiency and are not easily adaptable to large-scale industrial procedures. For the synthesis of other polyhalogenated pyridines the authors further suggest starting from 2-fluoropyridine or 3-fluoropyridine. However, to introduce a fluorine atom the authors suggest the metalation of a suitable precursor compound and subsequent chlorination of the intermediate organometallic species before subjecting the chloro-compound to a chlorine/fluorine displacement process. Ekkert et al. (Organometallics 2014, 33, 7027-7030) report on the synthesis of compound III via rhodium catalyzed hydro defluorination of 2,3,5,6-tetrafluoropyridine, albeit with poor yields.

Starting from 3-halopyridine, the chlorination process of the present invention offers a more versatile approach towards compounds II and III than those given in the prior art above. It uses molecular chlorine, thus making the process accessable to industrial processes.

Direct chlorination of the pyridine core using molecular chlorine was described in literature. For example, thermal procedures were developed for the chlorination of (unsubstituted) pyridine in the vapor phase at temperatures of about 400°C yielding mixtures of 2-chloropyridine and 2,6-dichloropyridine in poor to moderate yields (see for example US 3899495). Processes at these temperatures reportedly suffer from the formation of side products, tarring and/or corrosion of the reaction vessels.

The authors in US 3297556 report on a process to overcome these disadvantages by employing photolytic conditions in the liquid or vapor phase. The use of UV light irradiation enabled the transformation at considerably lower temperatures (at about 80 to 125°C), providing 2-chloropyridine in poor to moderate yields.

With reference to these procedures US 5536376 teaches that photolytic conditions in the vapor phase might be employed at higher temperatures than previously reported, i.e. up to 300°C, provided the chlorine and/or pyridine is sufficiently diluted with water (molar ratio of pyridine:water is from 1:10 to 1:30) to prevent overheating, which causes unwanted side reactions. The authors explicitly state, however, that unwanted side reactions pose a problem above 300°C. In this manner the authors were able to obtain mixtures of 2-chloropyridine and 2,6-dichloropyridine in moderate yields.

From an economic point of view, it is desirable in industrial large-scale operations to utilize highly selective, high yielding, inexpensive, simple and robust processes. In view of this, it was an object of the present invention to overcome the disadvantages of the reported procedures, providing a more efficient process for the chlorination of electron-deficient pyridines with high space-time yield and avoiding the formation of side products.

The inventors unexpectedly found, that 3-halopyridines can be chlorinated in a regioselective fashion, with excellent conversion rates and high yields according to the process of the present invention. This finding is surprising because a skilled person would have anticipated a sluggish reaction with molecular chlorine considering the electron-deficient nature of the heteroaromatic core in compound I. The inventors also found that the process is high-yielding particularly at temperatures above 200°C, with minimal amounts of side-products formed. This is in sharp contrast to the teaching of the prior art, which asserts that under such conditions intolerable amounts of side products will be formed.

Accordingly, the present invention relates to a process for the chlorination of 3-halopyridines of formula I, 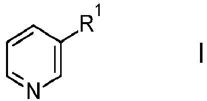 wherein R¹ is chlorine or fluorine;
the process comprising reacting compounds I in the presence of water and molecular chlorine under UV light irradiation and at a temperature above 200°C in the vapor phase to obtain compound of formula II

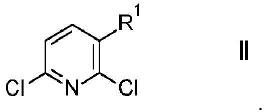

The general expression "compound I" as used herein is equivalent to the expression "compound of formula I".

The chlorination reaction is conducted in the presence of a light source. Generally radiant energy sources covering, at least to some extent, the range between 2000 and 6000 angstrom units are effective to bring about the chlorinations of the present invention. Suitable light sources are ultraviolet light, black fluorescent light, mercury vapor light.

The process of the present invention is typically carried out at atmospheric pressure. The reaction may also be conducted at elevated pressure, for example up to 2000 kPa.

In a preferred aspect of the process of the present invention, variable R¹ is fluorine.

In one aspect of the process of the present invention the temperature of the reaction mixture is above 220°C.

In one aspect of the process of the present invention the temperature of the reaction mixture is in the range between 220 and 400°C.

In yet another aspect the temperature of the reaction mixture is in the range between 220 and 350°C.

The presence of water in the reaction medium is essential because it prevents tarring. The inventors found, that the use of a larger excess of water reduces the yield of the transformation. Therefore, in one aspect of the process of the present invention, the amount of water is more than 1 and less than 30 equivalents, based on the amount of compound I.

In a preferred aspect the amount of water is more than 1 and less than 10 equivalents, based on the amount of compound I.

In still another preferred aspect the amount of water is more than 3 and less than 8 equivalents, based on the amount of compound I.

In one aspect of the process of the present invention the amount of molecular chlorine is 2-5 equivalents, based on the amount of compound I.

In a preferred aspect of the process of the present invention the amount of molecular chlorine is 2.0-3.5 equivalents, based on the amount of compound I.

In a preferred embodiment of the present invention the temperature of the reaction mixture is above 220°C; and the amount of molecular chlorine is 2-5 equivalents, based on the amount of compound I.

In a preferred embodiment of the present invention the temperature of the reaction mixture is in the range between 220 and 400°C; and the amount of molecular chlorine is 2-5 equivalents, based on the amount of compound I.

In another preferred embodiment of the present invention the temperature of the reaction mixture is above 220°C; and the amount of molecular chlorine is 2-5 equivalents, based on the amount of compound I; and the amount of water is more than 1 and less than 10 equivalents, based on the amount of compound I.

In another preferred embodiment of the present invention the temperature of the reaction mixture is in the range between 220 and 400°C; and the amount of molecular chlorine is 2-5 equivalents, based on the amount of compound I; and the amount of water is more than 1 and less than 10 equivalents, based on the amount of compound I.

In a preferred aspect of the present invention the temperature of the reaction mixture is in the range between 220 and 400°C; and the amount of molecular chlorine is 2.0-3.5 equivalents, based on the amount of compound I; and the amount of water is more than 3 and less than 8 equivalents, based on the amount of compound I.

In a preferred aspect of the present invention the temperature of the reaction mixture is in the range between 220 and 300°C; and the amount of molecular chlorine is 2.0-3.5 equivalents, based on the amount of compound I; and the amount of water is more than 1 and less than 10 equivalents, based on the amount of compound I.

In another preferred embodiment of the present invention the temperature of the reaction mixture is in the range between 220 and 300°C; and the amount of molecular chlorine is 2.0-3.5 equivalents, based on the amount of compound I; and the amount of water is more than 3 and less than 8 equivalents, based on the amount of compound I.

In one aspect the present invention relates to a process as defined above, further comprising the step of reacting compound II with a source of fluoride to obtain compound of formula III.

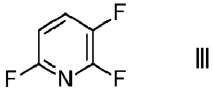

Typical sources of fluoride are , for example, potassium fluoride , potassium fluoride in mixture with hydrogen fluoride, sodium fluoride, calcium difluoride, Olah reagent (HF/pyridine), a polyhydrofluoride complex of a trialkylamine ((C₁-C₆-alkyl)₃N × n HF, wherein (n = 1-3) such as: (C₂H₅)₃N x 3 HF). Preferably, the fluorinating agent is used in an amount of 2.0 eq. to 10 eq., in particular 2.5-8.0 eq., more specifically 3.0-6.0 eq., based on the amount of compound II. According to a preferred embodiment the fluorinating reaction is carried out in the presence of an amine. Suitable amines are tertiary amines for example, tri(C₁-C₆-alkyl)amine such as trimethylamine, triethylamine or diisopropylethylamine, N-methylpiperidine, pyridine, substituted pyridines, such as 2,4,6-trimethylpyridine, 2,6-dimethylpyridine, 2-methylpyridine, 3-methylpyridine, 4-methylpyridine, and 4-dimethylaminopyridine; and also bicyclic amines such as 1,4-diazabicyclo[2.2.2]octane, 1,8-diazabicyclo[5.4.0]undec-7-ene, or 1,5-diaza-bicyclo[4.3.0]non-5-ene. Particular preference is given to using triethylamine, pyridine or 1,8-diazabicyclo[5.4.0]undec-7-ene.

In one aspect the fluorinating reaction is carried out in the presence of a phase transfer catalysts. Phase transfer catalysts suitable for use in the fluorination process are those well known in the art. Preferred phase transfer catalysts are selected from quaternary ammonium salts, quaternary pyridinium salts, quaternary phosphonium salts and any combination thereof and more preferably selected from quaternary ammonium salts, quaternary phosphonium salts and any combination thereof.

The fluorinating reaction is typically carried out in the presence of a polar solvent. The use of catalytic amounts of a polar solvent accelerates the fluorination reaction. Suitable organic solvents for the fluorination reaction are , aprotic polar solvents, for example: cyclic or acyclic ethers such as diethyl ether, diisopropyl ether, n-butyl methyl ether, isobutyl methyl ether, sec-butyl methyl ether, tert-butyl methyl ether, cyclopentyl methyl ether, methyl-tetrahydrofuran, tetrahydrofuran, 1,4-dioxane, ethylene glycol dimethyl ether, ethylene glycol, diethyl ether, diethylene glycol dimethyl ether, 2-methyltetrahydrofuran, glycol monomethyl- or monoethyl ether; cyclic or acyclic amides such as carboxamides (N,N-dimethylformamide, N,N-dimethylacetamide, N,N-diethylformamide, N,N-diethylacetamide, N-methylpyrrolidone, dimethyl imidazolinum, dimethylimidazolidinone, or tetramethylurea; or aliphatic nitriles such as acetonitrile or propionitrile, and mixtures of the aforementioned solvents. N-substituted lactams sulphoxides, sulphones (dimethyl sulfoxide, dimethyl sulfone, tetramethylene sulfoxide, tetramethylene sulfone, sulfolane). Preference is given to N,N-dimethylformamide, N-methylpyrrolidone , dimethylimidazolidinone , sulfolane. Preferably the polar solvent is sulfolane.

Typical reaction times for these fluorinations are in the range of from 1 to 24 hours, for example 4 to 12 hours. The reaction of the halogen exchange may be carried out at temperatures above 70°C, typically between 70°C and 180°C.

In another aspect the present invention relates to a process as defined above, further comprising the step of reacting compound III with aqueous or gaseous ammonia in a mixture with or without organic solvents like ethers, alcohols, dipolar aprotic solvents such as those defined above for fluorination reactions to obtain compound of formula IV.

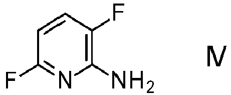

Aqueous ammonia is particularly preferred in industrial set-ups. It may be advantageous to conduct these reactions under pressure, for example in the range between 100 and 2000 kPa, preferably between 400 and 800 kPa, using at least stoichiometric amounts of ammonia. Typical reaction times for these aminations are in the range of from 1 to 24 hours, for example 4 to 8 hours. The reaction of the halogen exchange may be carried out at temperatures between 25 and 200°C, typically between 80°C and 150°C, preferably between 90 and 140°C.

In a further aspect the present invention relates to a process as defined above, further comprising the step of reacting compound IV with a chlorinating agent in a mixture with or without organic solvents like ethers, alcohols, dipolar aprotic solvents such as those defined above for fluorination reactions to obtain compound of formula V.

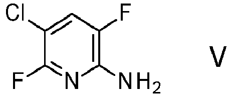

Suitable chlorination agents are N-chlorosuccinimide, trichloro isocyanuric acid in dipolar aprotic solvents, for example acetonitrile, N,N-dimethylformamide and N-methylpyrrolidine, at temperatures between -20 to 20°C.

### Working Examples

The present invention is further illustrated by the following working examples.

### Example 1) Chlorination of 3-fluoropyridine

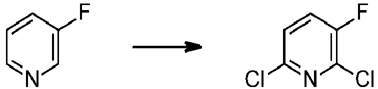

In a gas phase photochlorination tower equipped with 6 UV-lamps (800 W each, wavelength of 405 nm) 3-fluoropyridine (flow rate 20 kg/hour) was chlorinated with chlorine (flow rate 40 kg/hour) in the presence of water (flow rate 20 kg/hour) under irradiation of UV-light. 3-fluoropyridine and water were evaporated together at 180°C. The crude material was collected then heated to 60-70°C and the aqueous phase was separated. The organic phase was washed once with a 10% aqueous sodium hydroxide solution and twice with water. The water and low boiling components in the organic phase were removed by distillation (6-7 kPa, 100-120°C). The residue was used without further purification in next reaction step.

Table I summarizes the results obtained in accordance with the protocol of Example 1) under different reaction conditions.

**Table I:**

| Entry | Temperature of reaction mixture [°C] | Amount of chlorine based on amount of pyridine [equiv.] | Molar ratio pyridine : water | Isolated yield [%] |
|---|---|---|---|---|
| #1 | 240-260 | 2 | 1 : 6 | 76% |
| #2 | 280-310 | 2 | 1 : 6 | 86% |

### Example 2) Fluorination of 2,6-dichloro-3-fluoropyridine

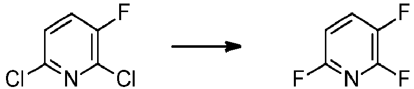

440 kg potassium fluoride (7.59 kmol, spray dried) and 40 kg tetraphenylphosphonium bromide (95.5 mol) was suspended in 2000 kg sulfolane (16.67 kmol). After that, sulfolane was distilled off under reduced pressure (3 kPa, 130°C) over 4 hours. After cooling to 25°C 500 kg 2,6-dichloro-3-fluoropyridine (2.47 kmol, purity 82.1wt-%) was added to the reaction mixture and stirred at 145°C for 4 hours and at 185°C for 12 hours. The reaction mixture was cooled to 60°C and 138.4 kg 2,3,6-trifluoropyridine (74.13wt-%), of a distillation fraction from a former pilot batch was added. Thereafter, the 2,3,6-trifluoropyridine was distilled off under reduced pressure, collected fractions yielded 93.4%.

### Example 3) Amination of 2,3,6-trifluoropyridine

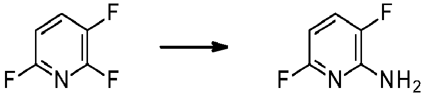

Aqueous ammonia (25%, 1134.5 kg, 16.684 kmol) and 452 kg 2,3,6-trifluoropyridine (89.4 wt-%, 3.038 kmol) were charged into a pressure reactor. The mixture was heated under stirring to 100-105°C for 6 hours. The mixture was cooled to 7-15°C and a white solid was precipitated. The slurry was filtered off and washed 4 times with cold water (485 kg, 7-15°C). The wet cake of 3,6-difluoro-pyridin-2-amine 382.6 kg (90.6 wt-%, yield 87.8%) was used without purification in the next step.

### Example 4) Chlorination of 3,6-difluoro-pyridin-2-amine

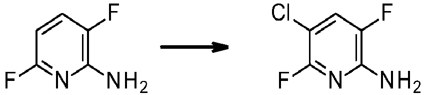

340 kg 3,6-difluoro-pyridin-2-amine (93.4 wt-%, 1.93 kmol) and 1301.5 Kg N,N-dimethylformamide were charged in a reactor. 206 kg trichloro cyanuric acid (95%) was dosed as powder into the mixture at -5°C over 15 hours. The reaction mixture was stirred at 0-5°C for additional 2 hours. After that, the reaction mixture was quenched by addition of a 20% aqueous solution of 254 kg sodium sulfite at ~0°C. Thereafter, 80% of the N,N-dimethylformamide (1041.2 kg) was distilled off at 40-50°C/ 95 kPa. To the remaining mixture a 5% aqueous solution of sodium hydroxide (3428 kg) was added and stirred for 30 minutes. After that, the product was filtered off and charged back to the reactor and treated again twice with 1714kg of a 5% solution of NaOH. The product was filtered off and dried in double cone rotating vacuum dryer for 50 hours (50°C, 95 kPa). 353.3 kg 5-chloro-3,6-difluoro-pyridin-2-amine (94.4 wt-%, yield 83.2%) was obtained after drying.

## Claims

1. A process for the chlorination of 3-halopyridines I, 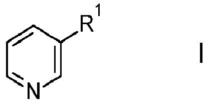 wherein R¹ is chlorine or fluorine;
the process comprising reacting compound I in the presence of water and molecular chlorine under UV light irradiation and at a temperature above 200°C in the vapor phase to obtain compound of formula II

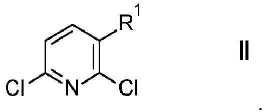

2. A process according to claim 1, wherein the temperature is in the range between 220 and 400°C.

3. A process according to claim 1 or 2, wherein the amount of chlorine is 2-5 equivalents, based on the amount of compound I.

4. A process according to any one of claims 1 to 3, wherein the amount of water is between 1 and 30 equivalents, based on the amount of compound I.

5. A process according to any one of claims 1 to 3, wherein the amount of water is more than 1 and less than 10 equivalents, based on the amount of compound I.

6. A process according to any one of claims 1 to 5, wherein R¹ is fluorine.

7. A process according to any one of claims 1 to 6, further comprising the step of reacting compound II with a source of fluoride to obtain compound of formula III

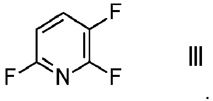

8. A process according to claim 7, further comprising the step of reacting compound III with ammonia to obtain compound of formula IV

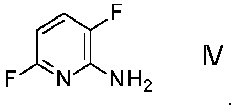

9. A process according to claim 8, further comprising the step of reacting compound IV with a chlorinating agent to obtain compound of formula V
